# EUROPEAN PATENT APPLICATION

(11) **EP 4 567 828 A1**
(43) Date of publication of application: **11.06.2025**
(21) Application number: 23214870.0
(22) Date of filing: 07.12.2023
(51) Int. Cl.: G16H 40/40

(54) **MONITORING MODEL ALERTS VOLUME CHANGES VIA EVOLUTION OF ALERTING PATTERNS**

(71) Applicant: Koninklijke Philips N.V., 5656 AG Eindhoven (NL)
(72) Inventor: POLIAKOV, Andrei, Eindhoven (NL); BHATTACHARYA, Sauvik, Eindhoven (NL); KOSTER, Robert Paul, 5656AG Eindhoven (NL)
(74) Representative: Philips Intellectual Property & Standards

(57) **Abstract**

The present invention relates to maintenance alert monitoring. In order to improve maintenance alert monitoring, there is provided an alert monitoring apparatus (10). The alert monitoring apparatus (10) comprises an input module (12), a change point analyzer (14), a classifier (16), an evolution analyzer (18), and an output module (20). The input module (12) is configured to receive a plurality of time series data of a maintenance alert from a plurality of electronic devices over a period of time, wherein each time series data represents a sequence of the maintenance alert over time from a respective electronic device. The change point analyzer (14) is configured to determine a sequence of alerting volumes over time based on the plurality of time series data of the maintenance alert, and to apply a change point detection method to the sequence of alerting volumes to divide the period of time into a succession of time segments, and to identify temporal boundaries of the time segments as change points. The classifier (16) is configured to classify each sequence of the maintenance alert within a given time segment of the succession of time segments into one of N classes, based on an occurrence pattern of the maintenance alert, wherein N is equal to or greater than 2. The evolution analyzer (18) is configured to determine a distribution of the classes among the plurality of electronic devices within each time segment, and to determine a change of the distribution of the classes over the succession of time segments. The output module (20) is configured to provide the determined change of the distribution of the classes over time.

## Description

### FIELD OF THE INVENTION

The present invention relates to maintenance alert monitoring, and in particular relates to an alert monitoring apparatus, to a decision-support system, to an alert monitoring method, and to a computer-readable storage medium.

### BACKGROUND OF THE INVENTION

Medical devices, such as patient monitoring devices and medical imaging devices, are used to collect various data, e.g., physiological data and image data, from a patient. Maintenance measures are inspections and servicing that are necessary to ensure the safe and proper operation of the medical devices on an ongoing basis. Maintenance information associated with the medical devices, such as usage and configuration information, may be collected, and maintenance alerts may be raised in case of e.g., security threats or misconfigurations in these medical devices. A technician may review the maintenance alerts associated with the medical devices and make decisions on the maintenance of the medical devices.

### SUMMARY OF THE INVENTION

There may be a need to improve maintenance alert monitoring.

The object of the present invention is solved by the subject-matter of the independent claims, wherein further embodiments are incorporated in the dependent claims.

According to a first aspect of the present invention, there is provided an alert monitoring apparatus, comprising:
- an input module configured to receive a plurality of time series data of a maintenance alert from a plurality of electronic devices over a period of time, wherein each time series data represents a sequence of the maintenance alert over time from a respective electronic device;
- a change point analyzer configured to determine a sequence of alerting volumes over time based on the plurality of time series data of the maintenance alert, and to apply a change point detection method to the sequence of alerting volumes to divide the period of time into a succession of time segments, and to identify temporal boundaries of the time segments as change points;
- a classifier configured to classify each sequence of the maintenance alert within a given time segment of the succession of time segments into one of N classes, based on an occurrence pattern of the maintenance alert, wherein N is equal to or greater than 2;
- an evolution analyzer configured to determine a distribution of the classes among the plurality of electronic devices within each time segment, and to determine a change of the distribution of the classes over the succession of time segments; and
- an output module configured to provide the determined change of the distribution of the classes over time.

Accordingly, instead of monitoring individual maintenance alerts, an alert monitoring apparatus is proposed to include the analysis of changes in alerting volume in the service and remediation workflow. As will be described in detail hereinbelow, the alert monitoring apparatus is proposed to classify the sequences of alerts over time per device, to track the qualitative behavior of changes in the sequences of maintenance alerts over the whole installed base with help of statistical data, and to monitor life cycle of the maintenance alerts based on the qualitative behavior. Different actions may be executed based on detected distribution of patterns. In one example, investigation of changes in alerting volume to monitor the health of the installed base may be sped up. In another example, in some cases unusual patterns may be treated as a marker of illegal user interventions. In a further example, unexpected deviations of a given device's life cycle from the majority of installations may be detected.

With the proposed alert monitoring apparatus, domain specific and qualitative changes in the maintenance alerts may be detected. For example, a sudden drastic increase in alerts for a specific category may represent a structural issue in the device installed base. The proposed alert monitoring apparatus may determine qualitative and quantitative metrics of the changes to facilitate the understanding of the nature of the changes, criticality of the issues affecting the devices, and act if needed. This may lead to decreased investigation and reaction time, efficient distribution of workforce, and, in the end, satisfaction of customers on the support quality.

Further, by tracking a distribution of the alert classes among the plurality of electronic devices, the proposed alert monitoring apparatus may identify the scale and extent of issues in parallel to the alerts themselves. Accordingly, service actions may be made more scalable. For example, it is possible to deploy a device upgrade in the entire installed base instead of individual alert follow up in case of structural issues being detected.

According to an exemplary embodiment of the first aspect of the present invention, the N classes comprise two or more of:
- a stable alert, wherein a sequence of the stable alert contains only a sequence of first values representing an occurrence of the maintenance alert over time with no second values or with expectation of a few second values in random places, wherein the second value represents no occurrence of the maintenance alert over time;
- a reoccurring alert, wherein a sequence of the reoccurring alert starts and ends with a sequence of first values representing an occurrence of the maintenance alert over time, and a sequence of second values therebetween representing no occurrence of the maintenance alert over time;
- a fixed alert, wherein a sequence of the fixed alert starts with a sequence of first values representing an occurrence of the maintenance alert over time and ends with a sequence of second values representing no occurrence of the maintenance alert over time; and
- a short-lived alert, wherein a sequence of the short-lived alert starts with a sequence of first values representing an occurrence of the maintenance alert over time and continues with a sequence of second values representing no occurrence of the maintenance alert over time, wherein the number of the first values is less than or equal to a predetermined number.

Accordingly, the sequences of maintenance alerts may be classified into one of the following classes/patterns: stable alerts, fixed alerts, reoccurring alerts, short-lived alerts.

According to an exemplary embodiment of the first aspect of the present invention, the evolution analyzer is configured to determine a change of the distribution of the classes over at least one change point, and to define the change of the distribution of the classes as one of a plurality of predefined evolution types.

Accordingly, the alert monitoring apparatus classifies the sequences of the maintenance alerts over time per device and tracks the evolution of alerts' life cycle over an install base. The evolution of alerts' life cycle may be determined based on the evolution of clusters with help of statistical data.

According to an exemplary embodiment of the first aspect of the present invention, the plurality of predefined evolution types comprise two or more of:
- a birth type representing that after the at least one change point, a new class of maintenance alert is identified compared with the classes identified within the time segment prior to the at least one change point;
- a death type representing that after the at least one change point, at least one of the classes identified within the time segment prior to the at least one change point disappears;
- a survive type representing that a majority of electronic devices from one class remain in the same class after the at least one change point;
- a split type representing that one or more electronic devices from one class identified within the time segment prior to the at least one change point become distributed among two or more classes after the at least one change point; and
- a merge type representing that one or more electronic devices from two or more classes identified within the time segment prior to the at least one change point merge into one class after the at least one change point.

Accordingly, after change point detection, also changes in e.g., daily alerting patterns for individual devices across the whole installed base may be determined.

According to an exemplary embodiment of the first aspect of the present invention, in the distribution of the classes, a weight of each class is represented by a percentage of electronic devices having the respective occurrence pattern of the maintenance alert.

Accordingly, the proposed alert monitoring apparatus may identify the scale and extent of issues in parallel to the alerts themselves. Accordingly, service actions may be made more scalable.

According to an exemplary embodiment of the first aspect of the present invention, the plurality of time series data of the maintenance alert is obtainable from time-stamped machine log data and/or time-stamped service log data for the plurality of electronic devices.

According to an exemplary embodiment of the first aspect of the present invention, the time-stamped machine log data includes log data used an electronic device and/or sensor data collected by a sensor.

According to an exemplary embodiment of the first aspect of the present invention, the plurality of the electronic devices comprises at least one medical device.

According to an exemplary embodiment of the first aspect of the present invention, the at least one medical device comprises one or more of a medical imaging device, and a patient monitoring device.

According to an exemplary embodiment of the first aspect of the present invention, the plurality of electronic devices comprises one or more of electronic devices belonging to a same category, electronic devices having a same modality, electronic devices used in a same geographical region, and electronic devices requiring a similar maintenance activity.

Accordingly, the proposed alert monitoring apparatus may be used to monitor domain specific issues in the device population health. For example, the proposed alert monitoring apparatus may be used to monitor a plurality of electronic devices designed with similar security model for a specific region.

According to an exemplary embodiment of the first aspect of the present invention, the alert monitoring device further comprises a maintenance analyzer configured to:
- detect an illegal user intervention based on the change of the distribution of the classes over time;
- determine an unexpected deviation of a life cycle of one or more electronic device from the life cycle of a majority of electronic devices based on the change of the distribution of the classes over time; and/or
- monitor the health of the plurality of electronic devices based on the determined change of alerting volume.

Accordingly, the proposed alert monitoring apparatus may determine qualitative and quantitative metrics of the changes to facilitate the understanding of the nature of the changes, and criticality of the issues affecting the devices. Different actions may be executed if needed.

According to an exemplary embodiment of the first aspect of the present invention, the alert monitoring device further comprises a network interface configured to connect the alert monitoring apparatus to a network.

Examples of the network interface may include, but are not limited to, Ethernet network interface, Wi-Fi network interface, 2G/3G network interface, LTE network interface, and 5G network interface.

According to a second aspect of the present invention, there is provided a decision-support system, comprising the alert monitoring apparatus according to the first aspect and any associated example to provide an alert monitoring result, and a web server configured to interface with a client device via a webpage and/or an application program served by the web server. The decision-support system is configured to provide a graphical user interface via the webpage and/or the application program to provide the alert monitoring result.

Accordingly, the alert monitoring apparatus may be a part of the decision-support system for remote monitoring engineers and modality performance managers.

According to a third aspect of the present invention, there is provided an alert monitoring method, comprising:
- receiving a plurality of time series data of a maintenance alert from a plurality of electronic devices over a period of time, wherein each time series data represents a sequence of the maintenance alert over time from a respective electronic device;
- determining a sequence of alerting volumes over time based on the plurality of time series data of the maintenance alert, and to apply a change point detection method to the sequence of alerting volumes to divide the period of time into a succession of time segments, and to identify temporal boundaries of the time segments as change points;
- classifying each sequence of the maintenance alert within a given time segment of the succession of time segments into one of N classes, on a basis of an occurrence pattern of the maintenance alert, wherein N is equal to or greater than 2;
- determining a distribution of the classes among the plurality of electronic devices within each time segment, and to determine a change of the distribution of the classes over the succession of the time segments; and
- providing the determined change of the distribution of the classes over time.

In this way, domain specific and qualitative changes in the maintenance alerts may be detected. This may lead to decreased investigation and reaction time, efficient distribution of workforce, and, in the end, satisfaction of customers on the support quality.

According to a further aspect of the present invention, there is provided a computer-readable storage medium comprising instructions which, when executed by a processor, cause the processor to carry out the steps of the method according to the third aspect and ay associated example.

It is noted that the above embodiments may be combined with each other irrespective of the aspect involved. Accordingly, the method may be combined with structural features of the device and/or system of the other aspects and, likewise, the device and/or the system may be combined with features of each other, and may also be combined with features described above with regard to the method.

These and other aspects of the invention will be apparent from and elucidated with reference to the embodiments described hereinafter.

### BRIEF DESCRIPTION OF THE DRAWINGS

These and other aspects of the invention will be apparent from and elucidated further with reference to the embodiments described by way of examples in the following description and with reference to the accompanying drawings, in which
FIG. 1 illustrates an exemplary system.
FIG. 2A illustrates an example of a sequence of alerting volumes over time over a period of time.
FIG. 2B illustrates the sequence of alerting volumes of FIG. 2A with divided time segments.
FIG. 3 illustrates a plurality of exemplary time series data of a maintenance alert from the plurality of electronic devices over a period of time.
FIG. 4 illustrates exemplary patterns of different alert classes.
FIG. 5 illustrates an exemplary pattern of different predefined evolution types.
FIG. 6 illustrates a further example of the system.
FIG. 7 illustrates a flow chart describing an alert monitoring method.

It should be noted that the figures are purely diagrammatic and not drawn to scale. In the figures, elements which correspond to elements already described may have the same reference numerals. Examples, embodiments or optional features, whether indicated as non-limiting or not, are not to be understood as limiting the invention as claimed.

### DETAILED DESCRIPTION OF EMBODIMENTS

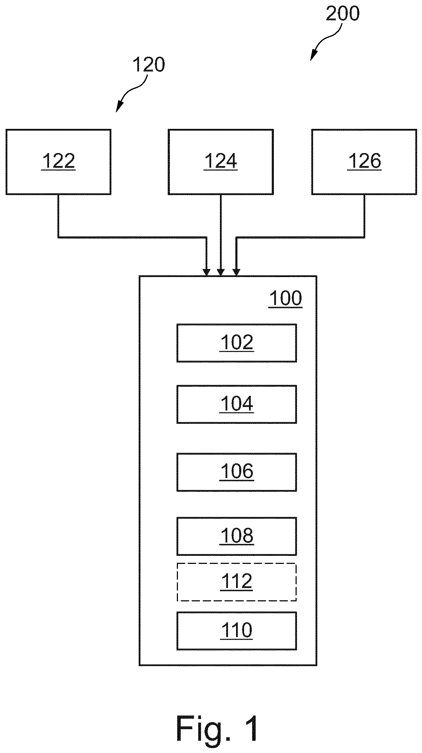

FIG. 1 illustrates an exemplary system 200 for electronic devices. In the illustrated example, the exemplary system 200 comprises a plurality of electronic devices 120 and an alert monitoring apparatus 100.

In the illustrated example shown in FIG. 1, the plurality of electronic devices 120 comprises a first electronic device 122, a second electronic device 124, and a third electronic device 126. While FIG. 1 may show a limited number of electronic devices by way of example, it will be appreciated that the plurality of electronic devices 120 may comprise a different number of electronic devices. The plurality of electronic devices 120 may include medical devices. In an example, the first electronic device 122, the second electronic device 124, and the third electronic device 126 may include one or more patient monitoring devices configured to collect physiological data from patients. In an example, the first electronic device 122, the second electronic device 124, and the third electronic device 126 may include one or more medical imaging devices to collect image data from a patient. Examples of the medical imaging devices may include, but are not limited to, X-ray imaging device, computed tomography (CT) imaging device, magnetic resonance (MR) imaging device, and ultrasonic imaging device. In some examples, the plurality of electronic devices 120 may also include one or more desktop, laptop, wall-mounted monitor devices, etc. In some examples, the plurality of electronic devices 120 may be spread throughout a facility, such as a clinic or hospital. In some other examples, the plurality of electronic devices may be located at different facilities spread out geographically. These electronic devices 120 need to be serviced at periodic intervals. Likewise, the software and firmware running on these electronic devices may need to be periodically updated. The plurality of time series data of the maintenance alert may be obtained from time-stamped machine log data and/or time-stamped service log data for the plurality of electronic devices. The time-stamped machine log data may include log data used an electronic device and/or sensor data collected by a sensor. For example, automated diagnostic monitoring models may be deployed to continuously scan these electronic devices, e.g., on a daily basis. For example, the diagnostic monitoring models may analyze data logged daily by each electronic device and raise alerts in case of security threats or misconfigurations in these electronic devices.

The alert monitoring apparatus 100 is coupled to the plurality of electronic devices 120 via a wired connection and/or a wireless connection, and collects the maintenance alerts from the plurality of electronic devices 120. In the illustrated example, the alert monitoring apparatus 100 comprises an input module 102, a change point analyzer 104, a classifier 106, an evolution analyzer 108, and an output module 110.

The alert monitoring apparatus 100 may be implemented in numerous ways (e.g., such as with dedicated hardware) to perform the functions described herein. A "processor" is one example of the alert monitoring apparatus 100, which employs one or more microprocessors that may be programmed using software to perform various functions described herein. The alert monitoring apparatus 100 may be implemented with or without employing a processor, and also may be implemented as a combination of dedicated hardware to perform some functions and a processor (e.g., one or more programmed microprocessors and associated circuitry) to perform other functions. Examples of alert monitoring apparatus components that may be employed in various embodiments of the present disclosure include, but are not limited to, conventional microprocessors, application specific integrated circuits (ASICs), and field-programmable gate arrays (FPGAs). In various implementations, a processor may be associated with one or more storage media (generically referred to herein as "memory," e.g., volatile and non-volatile computer memory). In some implementations, the storage media may be encoded with one or more programs that, when executed on one or more processors and/or controllers, perform at least some of the functions described herein. Various storage media may be fixed within the alert monitoring apparatus or may be transportable, such that the one or more programs stored thereon can be loaded into the alert monitoring apparatus so as to implement various aspects of the present disclosure described herein. The terms "program" or "computer program" are used herein in a generic sense to refer to any type of computer code (e.g., software) that can be employed to program one or more processors.

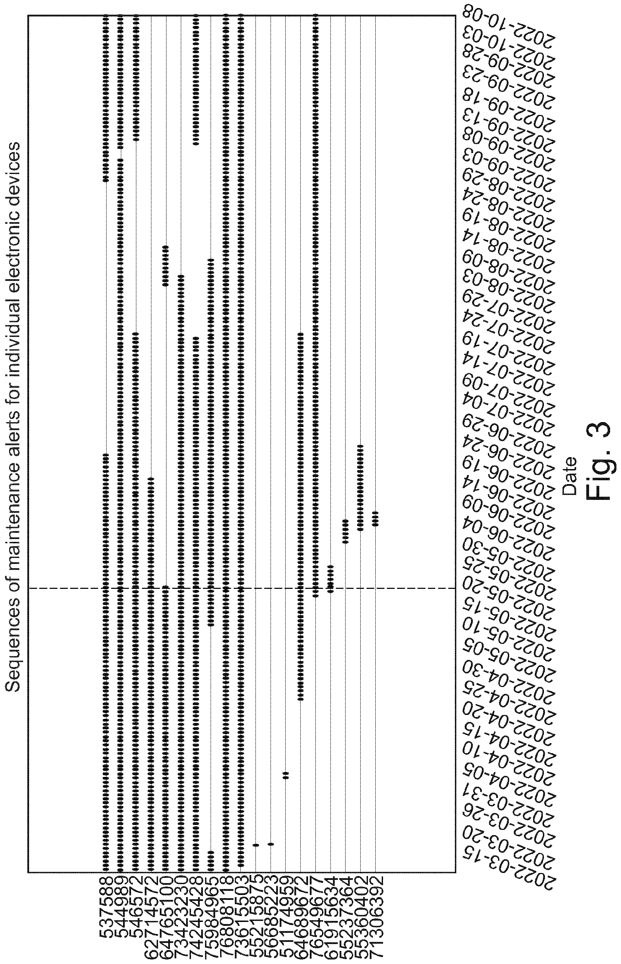

The input module 102 is configured to receive a plurality of time series data of a maintenance alert from the plurality of electronic devices 120 over a period of time. Each time series data represents a sequence of the maintenance alert over time from a respective electronic device. The sequence of maintenance alert may comprise a time stamp and a value associated with the time stamp. The value may comprise a first value representing an occurrence of the maintenance alert or a second value representing no occurrence of the maintenance alert. For example, the value of "1" may represent an occurrence of the maintenance alert, and a value of "0" may represent no occurrence of the maintenance alert. An example of the time series data is shown in FIG. 3 and will be explained in detail hereinafter.

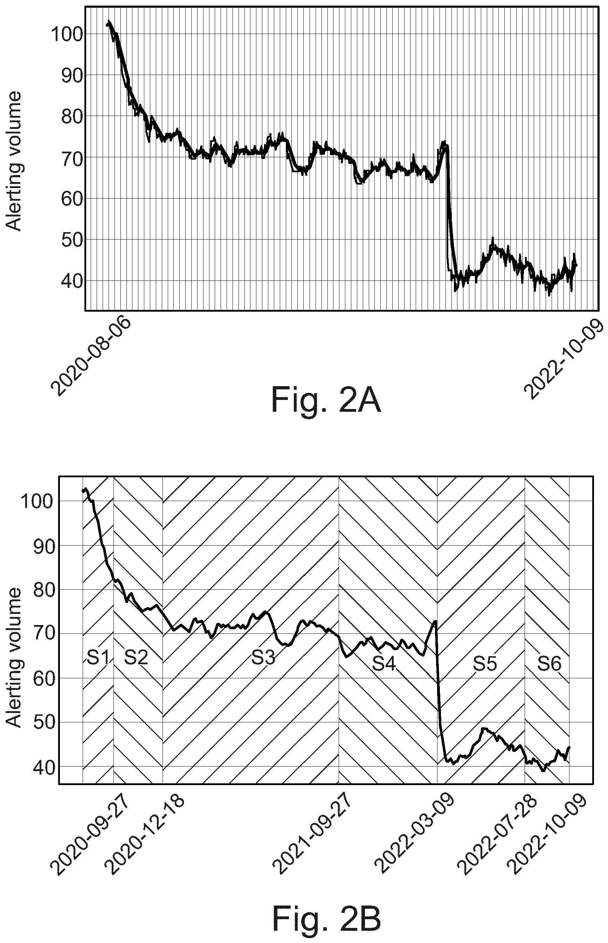

The change point analyzer 104 is configured to determine a sequence of alerting volumes over time based on the plurality of time series data of the maintenance alert. Each alerting volume in the sequence of alerting volume may represent the total number of maintenance values collected from the plurality of electronic devices per day. For example, FIG. 2A illustrates an example of a sequence of alerting volumes over time over a period of time between September 27, 2020 and October 9, 2022. In the diagram shown, the time stamp is shown on the X-axis, and the alerting volume, i.e., the total number of maintenance alerts collected from the plurality of electronic devices per day, is shown on the Y-axis.

The change point analyzer 104 is further configured to apply a change point detection method to the sequence of alerting volumes to divide the period of time into a succession of time segments. The values of alerting volumes within each segment may have a similar mean, standard deviation, linear trend, or count. The change point analyzer 104 may be configured to detect changes in mean, standard deviation, linear trend, and/or count. In one example, the change point analyzer 104 may be configured to detect shifts in the mean value of the alerting volumes. In this example, the mean value is constant within each segment and changes to a new value at each change point. In another example, the change point analyzer 104 may be configured to detect changes in the standard deviation of the alerting volumes. In this case, the standard deviation is constant within each segment and changes to a new value at each change point. In another example, the change point analyzer 104 may be configured to detect changes in the linear trend of the alerting volumes. In this example, the alerting volumes are assumed to follow a normal distribution with a mean value defined by a line. The slope and intercept of the line are constant within each segment and change to new values at each change point. In a further example, the change point analyzer 104 may be configured to detect changes in the mean value of the alerting volumes representing counts. The data values are assumed to follow a Poisson distribution within each segment with a mean that changes to a new value at each change point. The change point detection method may be an offline change point detection method, which assumes an existing time series with a start and end, and looks back in time to determine when changes occur.

FIG. 2B illustrates the sequence of alerting volumes of FIG. 2A with divided time segments. As shown in FIG. 2B, the change point detection method divides the time series into a succession of time segments, including segment S 1, segment S2, segment S3, segment S4, segment S5, and segment S6 shown in FIG. 2B. The values of alerting volumes within each segment may have a similar mean, standard deviation, linear trend, or count. The change point analyzer 104 is further configured to identify temporal boundaries of the time segments as change point. As an example, the change points may be defined as the first time step in each new segment starting with the second segment. In FIG. 2B, the following change points are identified: 2020-09-27, 2020-12-18, 2021-09-27, 2022-03-09, 2022-07-28, and 2022-10-09.

Turning back to FIG. 1, the classifier 106 is configured to classify each sequence of the maintenance alert within a given time segment of the succession of time segments into one of N classes, based on an occurrence pattern of the maintenance alert. N is equal to or greater than 2.

FIG. 3 illustrates a plurality of exemplary time series data of a maintenance alert from the plurality of electronic devices over a period of time. Each time series data represents an occurrence of a maintenance alert (e.g., security misconfiguration, threat, etc.) in the form of an "alert" plotted per unique electronic device over time. Each occurrence of the maintenance alert is represented by a dot. Each electronic device is represented by a unique system UID per horizontal row in the Y-axis, and the time stamps are shown in the X-axis. The maintenance alerts from a specific electronic device may be represented by values. It is possible to use a binary string with the value of "1" representing an occurrence of the maintenance alert, and the value of "0" representing no occurrence of the maintenance alert.

The classifier 106 may be configured to provide the classification of the maintenance alerts into two or more of the following classes: a stable alert, a reoccurring alert, a fixed alert, and a short-lived alert.

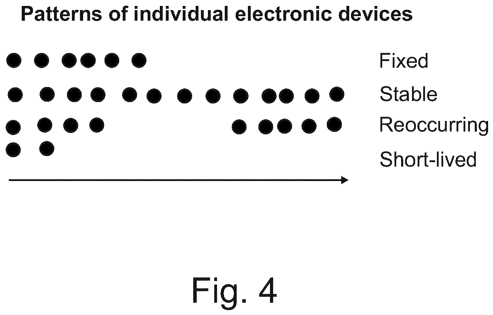

FIG. 4 illustrates some exemplary patterns of these classes.

The fixed alert comprises a sequence starting with a sequence of first values (e.g., "1") and ending with a sequence of the second values (e.g., "0"). In the exemplary fixed alert shown in FIG. 4, the sequence of the fixed alert starts with a sequence of ones and ends with zeros.

The stable alert comprises a sequence with only a sequence of the first values representing an occurrence of the maintenance alert over time with no second values or with expectation of a few second values in random places. For example, in the exemplary stable alert shown in FIG. 4, the sequence of the stable alert contains only ones with possible exception of a few zeros in random places.

The reoccurring alert comprises a sequence of starting and ending with a sequence of first values, and a sequence of the second values therebetween. In the exemplary reoccurring alert shown in FIG. 4, the sequence of maintenance alerts starts and ends with several ones and zeros in between.

The short-lived alert comprises a sequence of the short-lived alert starting with a sequence of the first values and continuing with a sequence of the second values. The number of the first values is less than or equal to a predetermined number. For example, in the exemplary short-lived alert shown in FIG. 4, the sequence of alerts is short-lived, if it contains only a few ones in a row somewhere in a middle of the string.

The classifier 106 may be a machine learning classifier, a deep learning classifier, or a rule-based classifier. The classifier 106 may classify all the sequences of maintenance alerts from the electronic devices within considered time-window into one of the above alert classes.

Turning back to FIG. 1, the evolution analyzer 108 is configured to determine a distribution of the classes among the plurality of electronic devices within each time segment, and to determine a change of the distribution of the classes over the succession of time segments. In other words, the evolution analyzer 108 tracks qualitative behavior of changes in sequences of alerts over the whole installed base.

For example, for every segment in timeline shown in FIG. 2B, the classifier 106 makes a classification of the maintenance alerts. The output of this procedure may be a multipartite graph, where number of parts equals to number of segments, and vertices are represented by classes: stable, reoccurring, short-lived, and fixed.

The weight of each class may be represented by percentage of electronic devices with this pattern within a given time-window. The edges of the graph may be defined by changes in distribution between the classes over time. The evolution analyzer 108 may be configured to define the change of the distribution of the classes as one of a plurality of predefined evolution types, such as birth type, death type, survive type, split type, and merge type.

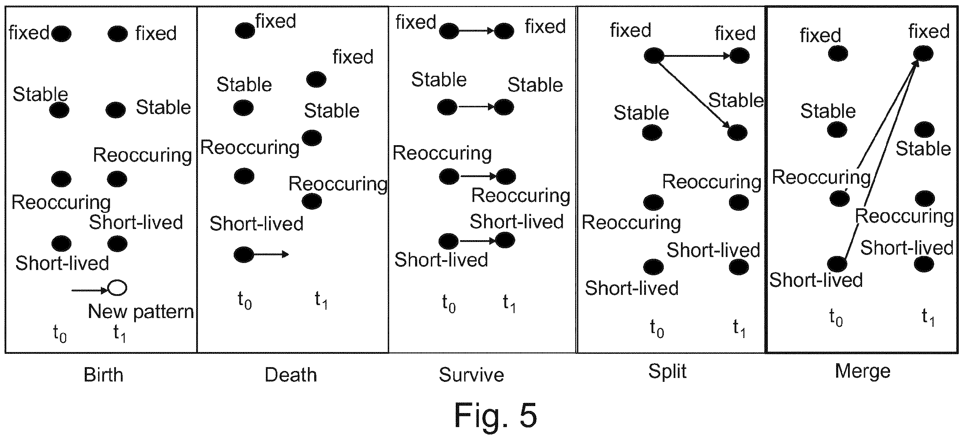

FIG. 5 illustrates an exemplary pattern of these predefined evolution types.

The birth type represents that after the at least one change point, a new class of maintenance alert is identified compared with the classes identified within the time segment prior to the at least one change point. For example, as show in FIG. 5, at the time point t0, there are four alert classes, namely fixed alert class, stable alert class, recurring alert class, and short-lived alert class. At the time point t1, there is a new pattern representing a new class in addition to the four classes.

The death type represents that after the at least one change point, at least one of the classes identified within the time segment prior to the at least one change point disappears. For example, as shown in FIG. 5, at the time point t0, there are four alert classes, namely, fixed alert class, stable alert class, recurring alert class, and short-lived alert class. At the time point t1, the short-lived alert class disappears.

The survive type represents that a majority of electronic devices from one class remain in the same class after the at least one change point. For example, as shown in FIG. 5, at the time point t0, there are four alert classes, namely, fixed alert class, stable alert class, recurring alert class, and short-lived alert class. At the time point t1, the four alert classes remain unchanged.

The split type represents that one or more electronic devices from one class identified within the time segment prior to the at least one change point become distributed among two or more classes after the at least one change point. For example, as shown in FIG. 5, at the time point t0, there are four alert classes, namely, fixed alert class, stable alert class, recurring alert class, and short-lived alert class. At the time point t1, the electronic devices from the fixed class becomes distributed among two alert classes, namely the fixed alert class, and the stable alert class.

The merge type represents that one or more electronic devices from two or more classes identified within the time segment prior to the at least one change point merge into one class after the at least one change point. For example, as shown in FIG. 5, at the time point t0, there are four alert classes, namely, fixed alert class, stable alert class, recurring alert class, and short-lived alert class. At the time point t1, some electronic devices from the reoccurring alert class and the short-lived alert class may be merged in to the fixed class.

Turning back to FIG. 1, optionally, the alert monitoring apparatus 100 may further comprise a maintenance analyzer 112. The maintenance analyzer 112 is configured to detect an illegal user intervention based on the change of the distribution of the classes over time, determine an unexpected deviation of a life cycle of one or more electronic device from the life cycle of a majority of electronic devices based on the change of the distribution of the classes over time, and/or monitor a health of the plurality of electronic devices based on the determined change of alerting volume. Accordingly, different actions may be executed based on detected distribution of patterns. In one example, investigation of changes in alerting volume to monitor the health of the installed base may be sped up. In another example, in some cases unusual patterns, e.g., short-lived or sporadic alerts, may be treated as a marker of illegal user interventions. In a further example, unexpected deviations of a given device's life cycle from the majority of installations may be detected.

The output module 110 is configured to output the determined change of the distribution of the classes over time. In some examples, the output module 110 may be configured to output the results on a user device. The user device may be a computer, a smartphone, a tablet, a smartwatch, a monitor, a data storage device, or any other device. In some examples, the output module 110 may be configured to output the results over time in a database. The database may be any organized collection of data, which can be stored and accessed electronically from a computer system. In some examples, the alert monitoring apparatus 100 may comprise a network interface configured to connect the alert monitoring apparatus to a network. The output module 110 may be configured to output the results to a remote server via the network interface. Examples of the network interface may include, but are not limited to, Ethernet network interface, Wi-Fi network interface, 2G/3G network interface, LTE network interface, and 5G network interface.

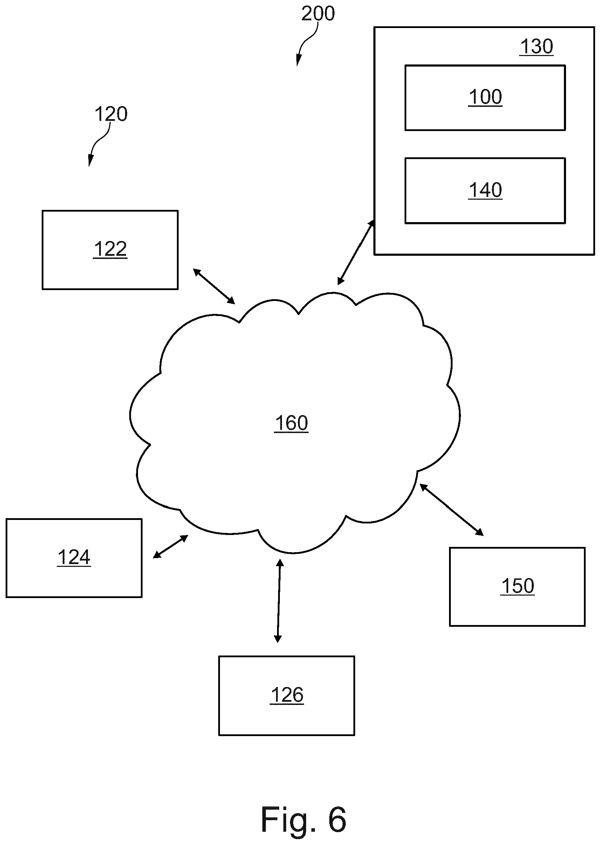

FIG. 6 illustrates a further example of the system 200. The exemplary system 200 comprises a plurality of electronic devices 120, a decision-support system 130, a service device 150, and a network 160.

In the illustrated example, the plurality of electronic devices 120 comprises a first electronic device 122, a second electronic device 124, and a third electronic device 126. These electronic devices can be located in a facility, such as a hospital or clinic. In one example, the electronic devices 122, 124, 126 may be located at the same facility. In another example, the electronic devices may be located at different facilities spread out geographically. The related content for the electronic devices is described above, and details are not described herein again.

The decision-support system 130 may be implemented as a server device which communicates through the network 160 with the plurality of electronic devices 120. For example, the decision-support system 130 may be located in the cloud, i.e., outside of the internal network associated with the first electronic device 122, the second electronic device 124, and the third electronic device 126. In this example, the decision-support system 130 may communicate with a central server located within the same network as the electronic devices 122, 124, 126. In some other examples, the decision-support system 130 may be located inside the internal network associated with the first electronic device 122, the second electronic device 124, and the third electronic device 126. For example, the decision-support system 130 may be a part of a central server located within the same network as the first electronic device 122, the second electronic device 124, and the third electronic device 126.

In this illustrated example, the alert monitoring apparatus 100 is embodied in the decision-support system 130 for monitoring the maintenance alerts received from the plurality of electronic devices 120. In other words, the alert monitoring apparatus may be a part of decision support system 130 for remote monitoring engineers and modality performance managers. The related content for the alert monitoring apparatus 100 is described above, and details are not described herein again.

The decision-support system 130 further comprises a web server 140 configured to interface with the service device 150 via a webpage and/or an application program served by the web server 140. The decision-support system 130 is configured to provide a graphical user interface via the webpage and/or the application program to provide the alert monitoring result. The web server 140 of FIG. 6 is a server that provides a web service to facilitate a user of the service device 150 to access the decision-support system 130. The web server 140 may interface with the user via webpages and/or application programs served by the web server 140 to facilitate the monitoring of the maintenance alerts. The web server 140 may, alternatively, be replaced with another device (e.g., another computing device) that provide any type of interface (e.g., a command line interface, a graphical user interface, etc.). In some systems, the alert monitoring apparatus 100 may include an integrated web server unit. In some other examples, a separate webserver 140 may be included.

The service device 150 of the illustrated example is used by a user (e.g., a technician) to access the maintenance information on the decision-support system 130. In some examples, the service device 150 may be a mobile device, such as a cellular telephone. Alternatively, the service device 150 may be any type of electronic device that is capable of communicating with the decision-support system 130 to access and manage maintenance information. Further examples of the service device 150 may be a personal computer (PC), or a workstation. The service device 150 may be a computing device. Alternatively, the service device 150 may be a device that typically does not include processing capabilities. For example, the service device 150 may be a simple user interface, e.g., touch screen.

The network 160 of the illustrated example communicatively couples the plurality of electronic devices 120, the decision-support system 130, and the service device 150. The network 160 is the internet. The network 160 may alternatively be any other type and number of networks. For example, the network 160 may be implemented by several local area networks connected to a wide area network. For example, the network 160 may comprise any combination of wired networks, wireless networks, wide area networks, local area networks, etc.

In operation, the decision-support system 130 may retrieve time series data of the maintenance alert from time-stamped machine log data and/or time-stamped service log data for the plurality of electronic devices 120, such as the first electronic device 122, the second electronic device 124, and the third electronic device 126, and transfer the retrieved time series data to the alert monitoring apparatus 100. The alert monitoring apparatus 100 determines changes in daily alerting patterns for individual devices across the whole installed base according to the approach described herein. The decision-support system 130 may provide a graphical user interface via the webpage and/or the application program to provide the alert monitoring result. For example, the graphical user interface may contain a dashboard for remote service or monitoring engineers and/or modality performance managers. It aims to visualize the evolution of changes between classes of alerting machines with respect to a maintenance alert.

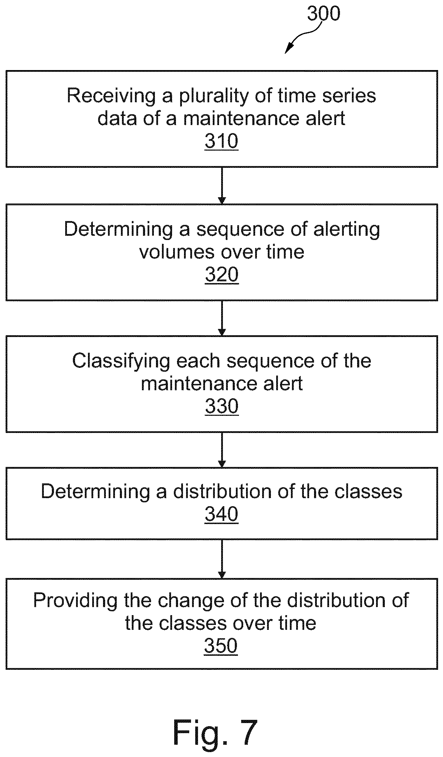

FIG. 7 illustrates a flow chart describing an alert monitoring method 300.

At block 310, the method 300 comprises the step of receiving a plurality of time series data of a maintenance alert from a plurality of electronic devices over a period of time. Each time series data represents a sequence of the maintenance alert over time from a respective electronic device. The time series data may comprise a time stamp and a value associated with the time stamp.

At block 320, the method 300 further comprises the step of determining a sequence of alerting volumes over time based on the plurality of time series data of the maintenance alert, applying a change point detection method to the sequence of alerting volumes to divide the period of time into a succession of time segments, and identifying temporal boundaries of the time segments as change points.

At block 330, the method 300 further comprises the step of classifying each sequence of the maintenance alert within a given time segment of the succession of time segments into one of N classes, on a basis of an occurrence pattern of the maintenance alert. N is equal to or greater than 2.

At block 340, the method 300 further comprises the step of determining a distribution of the classes among the plurality of electronic devices within each time segment, and determining a change of the distribution of the classes over the succession of the time segments.

At block 350, the method 300 further comprises the step of outputting the determined change of the distribution of the classes over time.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention. This exemplary embodiment of the invention covers both, a computer program that right from the beginning uses the invention and a computer program that by means of an up-date turns an existing program into a program that uses the invention. Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section. A computer program may be stored and/or distributed on a suitable medium, such as an optical storage medium or a solid state medium supplied together with or as part of other hardware, but may also be distributed in other forms, such as via the internet or other wired or wireless telecommunication systems. However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. An alert monitoring apparatus (10), comprising:
- an input module (12) configured to receive a plurality of time series data of a maintenance alert from a plurality of electronic devices over a period of time, wherein each time series data represents a sequence of the maintenance alert over time from a respective electronic device;
- a change point analyzer (14) configured to determine a sequence of alerting volumes over time based on the plurality of time series data of the maintenance alert, and to apply a change point detection method to the sequence of alerting volumes to divide the period of time into a succession of time segments, and to identify temporal boundaries of the time segments as change points;
- a classifier (16) configured to classify each sequence of the maintenance alert within a given time segment of the succession of time segments into one of N classes, based on an occurrence pattern of the maintenance alert, wherein N is equal to or greater than 2;
- an evolution analyzer (18) configured to determine a distribution of the classes among the plurality of electronic devices within each time segment, and to determine a change of the distribution of the classes over the succession of time segments; and
- an output module (20) configured to provide the determined change of the distribution of the classes over time.

2. The alert monitoring apparatus according to claim 1,
wherein the N classes comprise two or more of:
- a stable alert, wherein a sequence of the stable alert contains only a sequence of first values representing an occurrence of the maintenance alert over time with no second values or with expectation of a few second values in random places, wherein the second value represents no occurrence of the maintenance alert over time;
- a reoccurring alert, wherein a sequence of the reoccurring alert starts and ends with a sequence of first values representing an occurrence of the maintenance alert over time, and a sequence of second values therebetween representing no occurrence of the maintenance alert over time;
- a fixed alert, wherein a sequence of the fixed alert starts with a sequence of first values representing an occurrence of the maintenance alert over time and ends with a sequence of second values representing no occurrence of the maintenance alert over time; and
- a short-lived alert, wherein a sequence of the short-lived alert starts with a sequence of first values representing an occurrence of the maintenance alert over time and continues with a sequence of second values representing no occurrence of the maintenance alert over time, wherein the number of the first values is less than or equal to a predetermined number.

3. The alert monitoring apparatus according to claim 1 or 2,
wherein the evolution analyzer is configured to determine a change of the distribution of the classes over at least one change point, and to define the change of the distribution of the classes as one of a plurality of predefined evolution types.

4. The alert monitoring apparatus according to claim 3,
wherein the plurality of predefined evolution types comprise two or more of:
- a birth type representing that after the at least one change point, a new class of maintenance alert is identified compared with the classes identified within the time segment prior to the at least one change point;
- a death type representing that after the at least one change point, at least one of the classes identified within the time segment prior to the at least one change point disappears;
- a survive type representing that a majority of electronic devices from one class remain in the same class after the at least one change point;
- a split type representing that one or more electronic devices from one class identified within the time segment prior to the at least one change point become distributed among two or more classes after the at least one change point; and
- a merge type representing that one or more electronic devices from two or more classes identified within the time segment prior to the at least one change point merge into one class after the at least one change point.

5. The alert monitoring apparatus according to any one of the preceding claims,
wherein in the distribution of the classes, a weight of each class is represented by a percentage of electronic devices having the respective occurrence pattern of the maintenance alert.

6. The alert monitoring apparatus according to any one of the preceding claims,
wherein the plurality of time series data of the maintenance alert is obtainable from time-stamped machine log data and/or time-stamped service log data for the plurality of electronic devices.

7. The alert monitoring apparatus according to claim 6,
wherein the time-stamped machine log data includes log data used an electronic device and/or sensor data collected by a sensor.

8. The alert monitoring apparatus according to any one of the preceding claims,
wherein the plurality of the electronic devices comprises at least one medical device.

9. The alert monitoring apparatus according to claim 8,
wherein the at least one medical device comprises one or more of:
- a medical imaging device; and
- a patient monitoring device.

10. The alert monitoring apparatus according to any one of the preceding claims,
wherein the plurality of electronic devices comprises one or more of:
- electronic devices belonging to a same category;
- electronic devices having a same modality;
- electronic devices used in a same geographical region; and
- electronic devices requiring a similar maintenance activity.

11. The alert monitoring apparatus according to any one of the preceding claims, further comprising a maintenance analyzer configured to:
- detect an illegal user intervention based on the change of the distribution of the classes over time;
- determine an unexpected deviation of a life cycle of one or more electronic device from the life cycle of a majority of electronic devices based on the change of the distribution of the classes over time; and/or
- monitor a health of the plurality of electronic devices based on the determined change of alerting volume.

12. The alert monitoring apparatus according to any one of the preceding claims, further comprising:
- a network interface configured to connect the alert monitoring apparatus to a network.

13. A decision-support system (130), comprising:
- the alert monitoring apparatus (100) according to any one of the preceding claims to provide an alert monitoring result; and
- a web server (140) configured to interface with a client device via a webpage and/or an application program served by the web server;
wherein the decision-support system is configured to provide a graphical user interface via the webpage and/or the application program to provide the alert monitoring result.

14. An alert monitoring method (300), comprising:
- receiving (310) a plurality of time series data of a maintenance alert from a plurality of electronic devices over a period of time, wherein each time series data represents a sequence of the maintenance alert over time from a respective electronic device;
- determining (320) a sequence of alerting volumes over time based on the plurality of time series data of the maintenance alert, and to apply a change point detection method to the sequence of alerting volumes to divide the period of time into a succession of time segments, and to identify temporal boundaries of the time segments as change points;
- classifying (330) each sequence of the maintenance alert within a given time segment of the succession of time segments into one of N classes, on a basis of an occurrence pattern of the maintenance alert, wherein N is equal to or greater than 2;
- determining (340) a distribution of the classes among the plurality of electronic devices within each time segment, and to determine a change of the distribution of the classes over the succession of the time segments; and
- providing (350) the determined change of the distribution of the classes over time.

15. A computer-readable storage medium comprising instructions which, when executed by a processor, cause the processor to carry out the steps of the method of claim 14.
